# FASCICULE DE BREVET EUROPEEN

(11) **EP 2 714 875 B1**
(45) Date de publication et mention de la délivrance du brevet: **29.11.2017**
(21) Numéro de dépôt: 12731047.2
(22) Date de dépôt: 23.05.2012
(51) Int. Cl.: C11B 1/04, C11C 3/00

(54) **PROCÉDÉ DE TRITURATION RÉACTIVE DIRECTEMENT SUR UN TOURTEAU GRAS**
VERFAHREN FÜR REAKTIVE ZERKLEINERUNG DIREKT AUF EINEM ÖLKUCHEN
PROCESS OF REACTIVE TRITURATION DIRECTLY ON AN OIL CAKE

(30) Priorité: 25.05.2011 FR 1154554
(43) Date de publication de la demande: 09.04.2014
(73) Titulaire: Arkema France, 92700 Colombes (FR)
(72) Inventeur: DUBOIS, Jean-Luc, F-69390 Millery (FR); PICCIRILLI, Antoine, F-86000 Poitiers (FR)
(74) Mandataire: Hérard, Elise
(86) Numéro de dépôt international: PCT/FR2012/051159
(87) Numéro de publication internationale: WO 2012/160314

(56) Documents cités:
- WO-A1-2007/049979
- WO-A1-2007/130346
- US-A1- 2006 069 274
- US-A1- 2007 099 278

## Description

La présente invention porte sur un procédé de trituration réactive d'un tourteau gras, pour en extraire des esters d'acide gras.

Le « tourteau gras » est un résidu solide généralement issu d'un procédé de pressage de graines de plantes oléagineuses pour récupérer de l'huile (appelée huile de première pression à froid ou à chaud). Le tourteau peut aussi être issu d'un procédé dit de « double pression », comprenant une première pression des graines oléagineuses suivie d'une seconde étape de pression du tourteau produit au cours de l'étape précédente. Le tourteau gras peut contenir de 3 à 30% d'huile.

L'huile récupérée des graines de plantes oléagineuses est à usage alimentaire, énergétique ou industriel. Le pressage des graines de plantes oléagineuses, telles que le colza et le tournesol, s'est notamment développé pour disposer d'huile à usage de carburant.

Cette production d'huile s'accompagne d'une production importante de tourteaux gras qu'il est nécessaire de valoriser au mieux, afin d'optimiser le bilan économique global de cette transformation.

Certains tourteaux gras, de colza et de soja par exemple, peuvent présenter un intérêt en élevage pour l'alimentation animale. Néanmoins, on utilise généralement les tourteaux « déshuilés », contenant moins de 3% d'huile. Leur valeur nutritionnelle est plus intéressante, leur teneur en protéine est supérieure et celle en matière grasse plus faible que pour les tourteaux gras. De plus, les tourteaux gras ont tendance à rancir ce qui diminue l'appétence de l'animal pour ce type d'aliment.

Il est connu d'extraire l'huile restante des tourteaux gras, par percolation à contre-courant d'un solvant (comme l'hexane) chauffé à 40-60 °C pendant 4 à 5 heures. Il faut ensuite distiller le mélange d'huile et de solvant pour les séparer par chauffage à 115-120 °C sous aspiration et injection de vapeur. Les tourteaux ainsi « déshuilés » contiennent 0,5% à 2,5% d'huile. Mais les étapes de ce procédé d'extraction de l'huile par solvant sont coûteuses en énergie. Par ailleurs, compte tenu du caractère hautement volatil et inflammable de l'hexane, ces étapes sont classées comme dangereuses et conduisent à une libération importante de composés organiques volatils dans l'environnement. L'huile extraite du tourteau est de qualité médiocre comparativement à l'huile de première pression à froid et elle doit être raffinée. Les huiles extraites à l'hexane sont en effet riches en composés indésirables, tels que les phospholipides. De plus l'acidité de l'huile extraite est élevée, proche de celle des tourteaux gras de départ, qui ont eux-mêmes tendance à concentrer des acides gras libres. Cette acidité de l'huile a pour inconvénients de conduire à la production de savons lors des opérations de transestérification pour la production de biodiésel, mais aussi de réduire la stabilité à froid de l'huile. La présence d'acides gras libres complique aussi le procédé de raffinage.

Les huiles ainsi extraites et raffinées sont pour certaines comestibles et pour d'autres seulement utilisées en peinture, en savonnerie (par saponification de l'huile), en pharmacie, cosmétique, dans l'industrie, en oléochimie pour la production d'acides gras, etc.

Par transestérification de l'huile en présence d'alcool et de catalyseur, on fabrique en particulier des esters d'acides gras utilisés dans les biocarburants. Selon les techniques connues de l'art antérieur, la fabrication de ces esters à partir de tourteaux gras nécessiterait donc au moins deux étapes principales : extraction d'huile du tourteau puis transestérification, elles mêmes décomposées en de nombreuses sous-étapes.

La présente invention a donc pour but de fournir un nouveau procédé plus simple, présentant le moins d'étapes possibles, pour valoriser les tourteaux gras.

La demanderesse a maintenant trouvé un tel procédé permettant de fabriquer en une seule étape des esters d'acide gras à partir des tourteaux gras.

La présente invention a donc pour objet un procédé comprenant au moins une étape v) de trituration réactive consistant à mettre en contact un tourteau gras comprenant de 3 à 30% d'huile avec un alcool léger anhydre et un catalyseur alcalin dans des conditions de température et de durée suffisantes pour permettre l'extraction et la transestérification de l'huile végétale et conduisant à l'obtention d'un mélange comprenant des esters d'acides gras et du glycérol, et un tourteau déshuilé comprenant moins de 3% d'huile.

Par « alcool léger », on entend un alcool aliphatique inférieur dont le nombre de carbones est compris dans la gamme de 1 à 8, de préférence de 1 à 5, voire mieux de 1 à 4. L'alcool léger est avantageusement choisi parmi le méthanol, l'éthanol, l'isopropanol, le n-propanol, le butanol, l'isobutanol, le 2-ethylhexanol, et leurs mélanges. Selon un mode de réalisation préféré de l'invention, l'alcool léger est le méthanol.

Le catalyseur alcalin mis en oeuvre dans le procédé est choisi dans le groupe: soude, soude alcoolique, soude solide, potasse, potasse alcoolique, potasse solide, méthylate de sodium ou de potassium, éthylate de sodium ou de potassium, propylate de sodium et de potassium, isopropylate de sodium et de potassium. Selon un mode de réalisation préféré de l'invention, le catalyseur alcalin est la soude.

Le tourteau gras utilisé dans le procédé de l'invention est issu des graines de tout type de plantes oléagineuses : amande, arachide, carthame, chanvre, colza, coprah (noix de coco), coton, karité, lin, maïs, moutarde, navette, noix, olive, palmiste, pavot, ricin, jatropha, lesquerella, crambe, cameline, sésame, soja et tournesol en sont des exemples.

On ne sortirait pas du cadre de l'invention lorsque les tourteaux mis en oeuvre dans le procédé selon l'invention proviennent en tout ou partie de plantes oléagineuses génétiquement modifiées.

De préférence, le tourteau gras présente un taux d'acidité compris dans la gamme allant de 1 à 10 mg KOH/g, de préférence de 1 à 8 mg KOH/g, de préférence de 1 à 6 mg KOH/g, ceci pour éviter les réactions de saponification qui diminuent le rendement en esters. Cette acidité du tourteau gras est en grande partie liée au conditionnement qu'a subi le tourteau après sa production.

Le tourteau gras est utilisé seul ou en mélanges avec des graines entières (avec leur coque), ces graines provenant de la même plante ou bien d'une autre plante oléagineuse, oléo-protéagineuse ou protéagineuse, lesdites graines ayant de préférence un taux d'acidité inférieur à 3 mg KOH/g. Ces mélanges comprennent de préférence au moins 50% en poids de tourteau gras sur le poids total du mélange. Ces mélanges ont plusieurs avantages. D'une part, ils permettent de diminuer le taux d'acidité global de la matière triturée, car les tourteaux ont tendance à être plus acides que les graines dont ils sont issus. Or, les taux d'acidité bas permettent d'améliorer le rendement en ester du procédé. D'autre part, l'ajout de graines avec leur coque permet de freiner l'effet lubrifiant des tourteaux gras et améliore la cohésion de la charge à traiter, en créant par compression une sorte de « bouchon » qui augmente la percolation et améliore donc aussi le rendement de la réaction de trituration. Les mélanges de tourteau et graines compressés peuvent notamment supporter facilement plusieurs cycles de percolation au cours de l'étape de trituration v). Ces mélanges tourteau-graines permettent d'augmenter la surface de contact pour une meilleure percolation du mélange alcool-catalyseur et donc une meilleure extraction des lipides et leur transformation consécutive en esters.

Par tourteau gras au sens de l'invention, on entend un tourteau qui contient 3 à 30% de matière grasse, voire plutôt 5 à 25%, de préférence 7 à 20% de matière grasse. Par opposition au « tourteau déshuilé » contenant moins de 3% de matière grasse, le tourteau gras utilisé dans la présente invention n'a pas subi d'extraction ni de lavage par un quelconque solvant.

Dans la présente description de l'invention, on utilise indépendamment les termes « huile » ou « matière grasse » pour désigner les lipides extraits ou non du tourteau.

La teneur en huile des tourteaux gras utilisés dans la présente invention varie en fonction des conditions de pressage et des graines utilisées. On distingue généralement les tourteaux gras « fermiers » et les tourteaux gras « industriels ». Le pressage à la ferme est effectué sans prétraitement de cuisson des graines, et les tourteaux gras ont un contenu en matière grasse qui varie de 12% à 30%. Dans le cas des installations semi-industrielles ou industrielles, le pressage produit des tourteaux moins gras, généralement selon l'un des deux procédés suivants. Dans le pressage discontinu ou continu à froid, les produits sont broyés, concassés ou aplatis. L'huile (surtout d'olive et de noix) est extraite par pressions successives à une température inférieure à 80°C. Le rendement est faible, les tourteaux conservant 6 à 12% de matières grasses. Dans le pressage continu à chaud, les graines sont préchauffées jusqu'à 90°C, les graines sont broyées ou aplaties et ensuite pressées dans une vis sans fin où la température atteindra jusqu'à 120 °C. Une autre variante industrielle consiste à réaliser une seconde «étape de pression sur le tourteau issu de la première pression des graines. Le rendement est amélioré. Il reste de 4 à 20% d'huile dans le tourteau, selon les graines et les installations.

Avantageusement, la réaction a lieu dans un réacteur à lit fixe. Selon un mode de réalisation, le réacteur à lit fixe est une colonne de percolation thermorégulée équipée d'une grille. Une pompe permet d'alimenter la colonne en mélange alcool-catalyseur basique. L'alcool et le catalyseur sont donc ajoutés simultanément dans le réacteur, qui est maintenu à une température allant de 30 à 75°C, de préférence inférieure ou égale à 70°C, de préférence inférieure à 45°C, de préférence environ égale à 40°C. Le rapport massique catalyseur/alcool/tourteau est de préférence compris dans la gamme 0,001 à 0,01/0,1 à 10/1, de préférence de 0,001 à 0,01/0,1 à 5/1, de préférence dans la gamme de 0,002 à 0,01/0,1 à 3/1, de manière encore plus préférée dans la gamme de 0,002 à 0,01/0,1 à 2/1.

En particulier, une teneur en catalyseur inférieure à 0,001, voire inférieure à 0,002, ne permet pas d'obtenir un rendement en ester suffisant (c'est-à-dire un rendement d'au moins 50%), ni une conversion suffisante des triglycérides, diglycérides et surtout des monoglycérides. A l'inverse une teneur supérieure à 0,01 entraîne une saponification et donc aussi un mauvais rendement en esters.

L'alimentation est réalisée en tête de lit; le liquide réactionnel percole alors à travers le lit puis est récupéré dans une réserve située en aval, sous le lit. Par pompage, le liquide est renvoyé en tête de lit pour diffuser à nouveau dans le lit. La durée du cycle de recirculation du mélange alcool/catalyseur est de 15 à 60 minutes, de préférence de 20 à 40 minutes. En fin de cycle, l'alimentation en liquide est stoppée. Une partie du liquide encore présent dans le tourteau imbibé est alors récupérée par simple égouttage pendant une durée de 10 à 20 minutes. Le liquide récupéré peut subir une étape de neutralisation par ajout d'acide, puis une étape d'évaporation de l'alcool, pour conduire à un mélange de phases consistant en une phase plus légère riche en esters et une phase plus dense riche en glycérol. Le mélange de phases est soumis à une étape de décantation (consistant par exemple en une décantation statique dans un ou plusieurs décanteurs en parallèle ou en série, décantation centrifuge, combinaison de décantation statique ou centrifuge), permettant d'obtenir une phase supérieure composée majoritairement d'esters d'acide gras (phase ester) et une phase inférieure composée majoritairement de glycérine et d'eau (phase glycérine).

La phase ester est ensuite soumise à une séquence de réactions chimiques et/ou de séparations/purifications visant la récupération des esters gras, comprenant de manière connue une étape de lavage à l'eau suivie d'une étape de séchage sous vide.

L'ester d'acide gras ainsi obtenu est destiné notamment à la préparation de biodiésel.

L'autre produit issu directement du procédé selon l'invention est le tourteau déshuilé. Le tourteau déshuilé selon l'invention contient moins de 3% d'huile, de préférence moins de 2%, ou mieux moins de 1% en poids d'huile, sur le poids du tourteau.

Le procédé selon l'invention peut sans difficulté être mis en oeuvre en continu à l'échelle industrielle, par exemple au moyen : d'un réacteur-extracteur à bande mobile fonctionnant en continu (type extracteur De Smet) ; d'un filtre rotatif, d'une centrifugeuse, d'un décanteur centrifuge. De préférence, on opère la trituration réactive avec le méthanol à contre-courant du tourteau, sur plusieurs étages consécutifs. On ajoute également dans le milieu de réaction un solvant organique (co-solvant) miscible ou non-miscible avec ledit alcool léger. Dans ce cas, l'étape v) de trituration comprend l'introduction simultanée dans un réacteur contenant ledit tourteau, de l'alcool léger anhydre, du catalyseur basique, et en outre de co-solvant. Ce dernier est sélectionné dans le groupe : hexane, heptane, benzène, bicyclohexyle, cyclohexane, décaline, décane, essence, éther de pétrole, kérosène, kerdane, gazole, pétrole lampant, Méthylcyclohexane, Naphta (Texsolve V), Skellite, Tetradécane, Texsolve (B, C, H, S, S-2, S-66, S-LO, V), le CO2 supercritique, le propane ou le butane pressurisés, les solvants naturels tels que les terpènes (limonène, alpha et béta pinène)), des éthers tels que le diméthyléther, le diéthyléther, des cétones tels que l'acétone et des mélanges de tous ces solvants.

De préférence, le rapport alcool léger / co-solvant est compris dans la gamme de 10/90 à 90/10.

Avantageusement, le procédé selon l'invention comprend en outre, avant l'étape v) de trituration, au moins une étape ii) de pressage de graines de plantes oléagineuses pour récupérer une partie de l'huile des graines et fabriquer ledit tourteau gras. Tous dispositifs et procédés connus pour cette étape de pressage sont envisageables, tels que ceux déjà décrits plus haut.

De manière optionnelle, l'étape ii) de pressage est précédée d'au moins une des étapes i) suivantes : concassage, décorticage, préchauffage et/ou séchage des graines.

Par « concassage » des graines, on entend une réduction grossière de la taille de la graine, laquelle en fonction de sa taille initiale peut être divisée en 2 ou plusieurs parties plus ou moins fines. Cette opération peut être réalisée par exemple sur un aplatisseur à rouleaux cannelés ou encore sur un broyeur à bec ou à couteaux.

Par « décorticage » des graines, on entend la séparation des constituants de la graine que sont l'amande et la pellicule qui l'entoure. Cette opération contribue à débarrasser la graine d'un constituant non huileux, la pellicule, permettant ainsi d'accroître simultanément la productivité en huile du procédé ainsi que la teneur en protéines du tourteau.

Par « préchauffage » des graines, on entend un traitement thermique à chaud à une température comprise dans la gamme de 50 à 90°C, de préférence de 70 à 80°C, de la graine permettant selon l'objectif recherché :
- d'accroître la fluidité de l'huile,
- d'accroître la plasticité de la graine à presser,
- de coaguler les protéines.

Par « séchage » des graines, on entend un chauffage de la graine à une température comprise dans la gamme de 60 à 140°C, en vue, selon l'objectif recherché :
- d'ajuster l'humidité de la graine entre 0,5 et 5%,
- de détruire certains micro-organismes (ex. salmonelles),
- de désactiver les enzymes thermosensibles (ex. lipases),
- de décomposer les substances toxiques thermolabiles.

Selon un mode de réalisation préféré de l'invention, pour faciliter la percolation du solvant (à base d'alcool léger anhydre et de catalyseur alcalin) à travers le maillage tridimensionnel du tourteau, le tourteau est « floconné » avant l'étape v) de trituration réactive. Cette étape de floconnage iii) consiste à écraser le tourteau pour déstructurer son réseau tridimensionnel sans évacuer de matière grasse du tourteau.

Avantageusement, le tourteau gras est séché (étape iv) de séchage) avant l'étape v) de trituration réactive, de préférence juste après sa fabrication, à l'issue de l'étape ii) par exemple dans un séchoir continu à air chaud, à une température comprise entre 60 et 140°C en vue d'atteindre un taux d'humidité relative inférieur ou égal à 2%. De préférence, il s'écoule, entre l'étape ii) de fabrication du tourteau gras et l'étape v) de trituration réactive, moins de 72 heures, de préférence moins de 48 heures, de préférence moins de 24 heures, de préférence moins de 12 heures. Avantageusement, les étapes du procédé selon l'invention sont réalisées en continu. Ces modes de réalisations préférés ont pour but d'effectuer l'étape v) de trituration sur un tourteau gras « fraîchement préparé », en évitant qu'il s'oxyde, qu'il s'hydrate et s'hydrolyse en formant des acides gras libres.

Selon un mode de réalisation avantageux du procédé de l'invention, le tourteau est préalablement floconné par simple aplatissage ou aplatissage multiple, puis séché avant d'être mis en oeuvre en trituration réactive réalisée en présence ou en absence d'un co-solvant.

Le procédé selon l'invention permet de faire réagir « in planta » l'alcool léger avec l'huile contenue au coeur du tourteau. Dans ce procédé, l'alcool joue à la fois le rôle de solvant et de réactif. Le procédé selon l'invention permet de passer directement du tourteau aux esters d'acides gras, et ce sans extraction préalable de l'huile, en évitant les étapes de raffinage, de purification et la production de sous-produits comme des terres de blanchiment normalement obtenues lors d'une étape de filtration, des gommes composées majoritairement de phospholipides, et/ou de savons issus du raffinage (neutralisation des acides gras libres).

Le procédé de trituration réactive de tourteaux gras selon l'invention conduit à l'obtention simultanée d'un tourteau déshuilé, de glycérol et d'esters d'acides gras. On obtient par exemple des esters de jatropha dans le cas d'un tourteau gras de jatropha, ou des esters de ricin dont de l'ester d'acide ricinoléique dans le cas d'un tourteau gras de ricin. L'ester d'acide ricinoléique est destiné principalement à la fabrication d'acide 11-amino undécanoïque, monomère constitutif du Rilsan® 11, qui est un polyamide aux propriétés physiques exceptionnelles, développé par la demanderesse. Ces esters conviennent également à la fabrication de biocarburants.

L'invention a notamment pour objet un mélange d'esters d'acides gras, notamment d'esters méthyliques d'acide gras, susceptible d'être obtenu par le procédé de l'invention, et présentant une teneur en tocophérols supérieure à 10 mg/100g. Ces antioxydants naturels présents dans les huiles végétales confèrent une meilleure stabilité et une meilleure résistance à l'oxydation du mélange selon l'invention. De plus, au contraire des esters issus d'une extraction classique à l'hexane, qui doivent être raffinés pour éviter la production de savons, les esters d'acide gras selon l'invention peuvent être utilisés directement, par exemple comme biocarburant, sans étape de raffinage préalable.

Selon une variante de réalisation, le tourteau déshuilé imbibé d'alcool est séché à l'étuve ventilée pendant 4h à une température inférieure ou égale à 200°C, de préférence inférieure ou égale à 150°C et encore plus préférentiellement inférieure ou égale à 120°C. Cette étape de séchage permet d'éliminer du tourteau le solvant (alcool) utilisé lors de l'extraction. Cette étape de séchage permet également de détruire les toxines et allergènes éventuels restant dans le tourteau.

Selon une autre variante de réalisation, le procédé selon l'invention ne comprend pas d'étape de séchage du tourteau à haute température (température supérieure à 120°C) ; selon les conditions mises en oeuvre, les éventuelles toxines et allergènes peuvent être inactivés grâce aux traitements physiques et/ou chimiques appliqués aux tourteaux gras au cours des étapes du procédé de l'invention décrites plus haut, de sorte que l'opération de séchage du tourteau à des températures élevées devient inutile. Dans ce cas, le procédé comprend seulement une étape de séchage du tourteau à des températures inférieures à 120°C, destinée à éliminer le solvant (alcool) utilisé lors de l'extraction, afin de permettre l'utilisation dudit tourteau dans l'alimentation animale.

Avantageusement, le procédé selon l'invention permet d'obtenir des tourteaux complètement déshuilés, qui gardent leur intégrité physique (cohésion, résistance mécanique), et qui sont détoxifiés et désallergénisés, dans lesquels les toxines et allergènes éventuels ont été inactivés.

Les esters de phorbol par exemple sont la principale source de toxicité des tourteaux de Jatropha. Cette famille de composés est connue pour ses effets biologiques néfastes chez l'homme et l'animal, notamment dans l'inflammation et la promotion des tumeurs. Avantageusement, le(s) tourteau(x) déshuilé(s) détoxifié(s) selon l'invention présente(nt) une teneur en esters de phorbol inférieure ou égale à 0,3 mg/g, de préférence inférieure à 0,2 mg/g.

Parmi les toxines inactivées, on peut également citer par exemple la ricine présente dans les tourteaux gras de ricin, ou encore la curcine présente dans les tourteaux gras de jatropha. Parmi les allergènes, on peut citer l'allergène CB-1A présent dans les tourteaux gras de ricin. Des dosages de toxines, comme la ricine, dans le tourteau avant et après déshuilage montrent que les meilleurs résultats de détoxification du tourteau sont obtenus dans le cas du procédé selon l'invention, grâce à la présence simultanée d'alcool léger et de catalyseur alcalin. Au contraire, les procédés d'extraction classiques (trituration non réactive) des tourteaux au solvant, à l'hexane et/ou au méthanol par exemple, laissent toujours de fortes teneurs en toxines dans le tourteau. Les meilleurs résultats de détoxification sont obtenus par trituration réactive selon l'invention utilisant du méthanol et de la soude. On remarque en particulier que l'alcool léger utilisé seul (sans co-solvant) en présence de catalyseur alcalin donne de meilleurs résultats de détoxification que les mélanges d'alcool léger avec co-solvant (hexane par exemple) toujours en présence de catalyseur alcalin, qui eux-mêmes donnent de meilleurs résultats de détoxification du tourteau que les procédés d'extraction classiques sans catalyseur alcalin. On pense que c'est le catalyseur alcalin utilisé dans le présent procédé qui entraîne l'effet remarquable de détoxification du tourteau. Cet effet est d'autant plus observé que le tourteau de départ mis en oeuvre dans le procédé de l'invention est moins acide. En effet, dans le cas de tourteaux gras acides (d'indice d'acide supérieur ou égal à 10 mg KOH/g), le catalyseur alcalin a plutôt tendance à neutraliser les acides gras libres du tourteau pour former des savons et ne peut pas participer pleinement à la détoxification du tourteau.

Les tourteaux détoxifiés selon l'invention présentent un intérêt nutritionnel et peuvent être directement utilisés dans l'alimentation animale, sans constituer un risque pour la santé des personnes qui les manipulent.

Hormis les applications alimentaires, les tourteaux déshuilés selon l'invention sont riches en azote et peuvent aussi être utilisés comme engrais ou comme fuel. Ils peuvent être utilisés comme charbon (après carbonisation du tourteau), éventuellement activé pour faire du charbon actif, comme charge végétale dans les matériaux composites biosourcés, comme bio-charbon utilisé dans l'agriculture, ou encore pour la fabrication de bio-gaz, comme source d'azote pour les microorganismes dans les réactions de méthanisation pour produire de l'énergie sous forme de méthane.

L'invention et ses avantages seront mieux compris à la lecture des exemples ci-après donnés à titre purement illustratif et non limitatif.

### Exemples

Sauf indication contraire, tous les pourcentages sont donnés en poids.

Dans les exemples ci-après, le procédé de trituration réactive selon l'invention est effectué sur des tourteaux issus d'une étape d'extraction par pression mécanique.

### Exemple 1 : Essai de trituration réactive sur un tourteau pression de jatropha Préparation du tourteau de jatropha

Avant la pression de la graine de jatropha sur une presse Taby 40A, la graine de jatropha est pré-concassée sur aplatisseur à rouleaux cannelés.

**Tableau 1 : Paramètres de la pression mécanique des graines de jatropha**

| **Matériel** | **Paramètres** | **Remarque** |
|---|---|---|
| Outil | Mono-vis | Presse Taby |
| Diamètre de la filière, mm | 12 | Filière semi-conique |
| Pré-chauffage graine | Oui | Température de 70 à 80 °C |

**Tableau 2 : Bilan-matière de la pression mécanique des graines de jatropha**

| **Matière entrante** | |
|---|---|
| Graines Jatropha double aplaties g | 6848 |
| Teneur en matières volatiles, % MB** | 7,5 |
| Teneur en matière grasse, % MS* | 35,0 |

| **Matière sortante** | |
|---|---|
| Tourteau gras, g | 4863 |
| Teneur en matières volatiles, %MB** | 9,0 |
| Teneur en matière grasse, %MS | **13,6** |
| Huile de pression brute non filtrée, kg | 1985,1 |

| | |
|---|---|
| *MS = Matière Sèche **MB = Matière brute | |

Le tourteau préparé présente une teneur en matière grasse de 13,6%. Ce dernier est immédiatement séché à 100°C pendant 16 heures.

### Exemple 2 : Essai de trituration réactive sur un tourteau pression de jatropha

La trituration réactive est réalisée dans une colonne de percolation équipée d'un lit fixe. Elle est mise en oeuvre dans les conditions suivantes :
1. Le tourteau brut tel que sorti de presse, de forme cylindrique (Longueur = 20 mm, diamètre = 12 mm), est séché à l'étuve à 100°C pendant 16 heures. Sa teneur en humidité relative est de 0,8%.
2. Le tourteau séché est introduit dans la colonne de percolation (350 g).
3. La solution de soude méthanolique à 0,4% par rapport au tourteau est alors recirculée sur le lit pendant 30 minutes à 50°C.
4. Le miscella est ensuite soutiré et le lit de flocon est ensuite lavé par 5 lavages successifs par du méthanol à 50°C (5 minutes par lavage).

Au vu des résultats du tableau 3, il s'avère que dans les conditions de tests, le rendement en ester est de 50%. Ce faible rendement est lié à une forte activité saponifiante (fort rendement en glycérine, supérieur à 100% en poids car il contient d'autres composés).

**Tableau 3 : Optimisation de la teneur en catalyseur sur le tourteau de jatropha**

| **ESSAI** | **10-E36** |
|---|---|
| Teneur en catalyseur (vs tourteau gras), % | **0,4** |
| Température de réaction et extraction | **50** |
| Rapport massique Méthanol / Tourteau gras | 1,6 |
| Rendement en extrait sec (1), % | 94,5 |
| Déphasage ester/glycérine | oui |
| Rendement en ester, % | **50,2** |
| Rendement en glycérine, % | 533 |
| Potentiel ester tourteau, % | **17,6** |
| Teneur en matière grasse du tourteau déshuilé, %MS* | 2,6 |
| Perte ester (2), % | 32,2 |

| | |
|---|---|
| (1) : Le rendement en extrait sec est le rapport fois 100 de l'extrait sec obtenu après évaporation du miscella sur la somme des quantités théoriques d'ester et de glycérine (2) Perte ester = 100 - Rdt Ester - Pot. Ester Tourteau *MS = Matière Sèche | |

Au plan qualitatif (tableau 4), l'ester produit avec 0,4% de catalyseur engagé, présente une acidité et une teneur en glycérides modérées.

**Tableau 4 : Bilan analytique des esters de jatropha**

| **Critère** | **10-E36** |
|---|---|
| Indice d'acide (mg KOH/g) | 0,5 |
| Teneur en MonoGlycéride (%) | 0,8 |
| Teneur en DiGlycéride + TriGlycéride (%) | 0,15 |

En présence de tourteau pression, lequel se présente sous la forme de pellets cylindriques, on constate que la vitesse de percolation de la solution de soude méthanolique apportée dans un rapport [méthanol / tourteau] de 1,6, est très importante.

C'est pourquoi, dans les essais suivants, on cherche à améliorer le temps de contact lipides-soude méthanolique ainsi que l'extractibilité de la matière grasse en diminuant le rapport [méthanol/tourteau], mais aussi en réalisant l'essai en présence de co-solvant (hexane ici).

### Exemple 3 : Adaptation des quantités de co-solvant et de catalyseur

La trituration réactive est réalisée dans une colonne de percolation équipée d'un lit fixe. Elle est mise en oeuvre dans les conditions suivantes :
1) Le tourteau brut tel que sorti de presse, de forme cylindrique (longueur = 20 mm, diamètre = 12 mm), est séché à l'étuve à 100°C pendant 16 heures. Sa teneur en humidité relative est de 0,8%.
2) Le tourteau séché est introduit dans la colonne de percolation (350 g).
3) La solution constituée de soude méthanolique à 0,27% (par rapport au tourteau) et d'hexane est alors recirculée sur le lit pendant 30 minutes à 40°C.
4) Le miscella est ensuite soutiré et le lit de flocon est ensuite lavé par 5 lavages successifs par un mélange de méthanol et d'hexane (dans un rapport massique 90/10 à 40°C (5 minutes par lavage).

Dans l'essai 10-E43 (tableau 5 suivant), la température de trituration réactive à été diminuée de 10°C (50→40°C) afin d'éviter l'évaporation de l'hexane. Dans les conditions de l'essai (conditions adaptées à la teneur en matière grasse (MG) du tourteau) et par comparaison à l'essai sans co-solvant, il apparaît que le rendement en esters du procédé selon l'invention est amélioré (50→58%), notamment grâce à la présence de co-solvant.

**Tableau 5 : Bilan matière de l'essai réalisé en présence de co-solvant**

| **ESSAI** | **10-E43** |
|---|---|
| Teneur en matière grasse, %MS* | **13,6** |
| Teneur en catalyseur (vs tourteau gras), % | **0,27** |
| Qualité MeOH/Hexane | **90/10** |
| Ratio MeOH/Hexane/ tourteau gras | 0,72/0,08/1 |
| Rendement en extrait sec (1), % | **85,0** |
| Déphasage ester/glycérine | Oui |
| Rendement en ester, % | **58,0** |
| Rendement en glycérine, % | 356 |
| Potentiel ester tourteau, % | **18,3** |
| Teneur en matière grasse du tourteau déshuilé, %MS* | **2,8** |
| Perte ester (2), % | 23,7 |

| | |
|---|---|
| (1) Le rendement en extrait sec est le rapport fois 100 de l'extrait sec obtenu après évaporation du miscella sur la somme des quantités théoriques d'ester et de glycérine (2) Perte ester = 100 - Rdt Ester - Pot. Ester Tourteau (3) MS* = Matière Sèche | |

**Tableau 6 : Bilan analytique des esters de jatropha**

| **Critère** | **Méthode** | **10-E43** |
|---|---|---|
| Indice d'acide (mg KOH/g) | EN14104 | nd |
| Teneur en MonoGlycéride (%) | ARKEMA | **2,4** |
| Teneur en Di et triglycérides (%) | ARKEMA | **3,9** |

### Exemple 4 : Adaptation des quantités de co-solvant et de catalyseur

Dans l'essai à suivre les quantités d'hexane et de catalyseur ont été augmentées pour accroître l'activité transestérifiante.

La trituration réactive est réalisée dans une colonne de percolation équipée d'un lit fixe. Elle est mise en oeuvre dans les conditions suivantes :
1) Le tourteau subit une opération préalable de simple aplatissage sur un aplatisseur équipé de rouleaux lisses (espacement de 0,05 mm) suivie d'un séchage à l'étuve à 100°C pendant 16 heures. Sa teneur en humidité relative est de 1,5%.
2) Le tourteau séché est alors introduit dans la colonne de percolation (350g).
3) La solution constituée de soude méthanolique à 0,7% (par rapport au tourteau) et d'hexane (mélange 50/50 massique) est alors recirculée sur le lit pendant 30 minutes à 40°C.
4) Le miscella est ensuite soutiré et le lit de flocon est ensuite lavé par 5 lavages successifs par un mélange de méthanol et d'hexane (dans un rapport massique 50/50 à 40°C (5 minutes par lavage).

Dans les conditions de l'essai 10-E51 (voir tableau 7 suivant), la température de trituration réactive est toujours de 40°C. Dans ces conditions, on constate que l'augmentation de la teneur en hexane et en catalyseur a un effet positif sur le rendement en esters (>80%) ainsi que sur l'épuisement du tourteau (0,2% de MG résiduelle).

**Tableau 7 : Bilan matière de l'essai de trituration réactive sur tourteau floconné réalisée à plus fortes teneurs en hexane et en catalyseur**

| ESSAI | 10-E51 |
|---|---|
| Teneur en matière grasse du tourteau n°2, %MS* | **18,7** |
| Acidité huile du tourteau, mg KOH/g | **6,0** |
| Teneur en catalyseur (vs tourteau gras), % | **0,7** |
| Ratio Méthanol/Hexane/ Tourteau gras (floconné) | 1/1/1 |
| Rendement en extrait sec (1), % | **110,4** |
| Déphasage ester/glycérine | Oui |
| Rendement en ester, % | **80,5** |
| Rendement en glycérine, % | **407,7** |
| Potentiel ester tourteau, % | **0,7** |
| Teneur en matière grasse du tourteau déshuilé, %MS* | **0,2** |
| Perte ester (2), % | **18,8** |

| | |
|---|---|
| (1) Le rendement en extrait sec est le rapport fois 100 de l'extrait sec obtenu après évaporation du miscella sur la somme des quantités théoriques d'ester et de glycérine (2) Perte ester = 100 - Rdt Ester - Pot. Ester Tourteau (3) MS* = Matière Sèche | |

Malgré l'acidité de la matière grasse du tourteau de départ initialement importante : 6 mg KOH/g, les esters produits selon le procédé de l'invention ont une acidité modérée avec un indice d'acide inférieur à 0,5 mg KOH/g (tableau 8).

**Tableau 8 : Bilan analytique des esters de jatropha**

| **Critère** | **Méthode** | **10-E51** |
|---|---|---|
| Indice d'acide (mg KOH/g) | EN14104 | **0,46** |
| Teneur en MonoGlycéride (%) | ARKEMA | **1,1** |
| Teneur en Di et triglycérides (%) | ARKEMA | 0,2 |

### Exemple 5 : détoxification des tourteaux de jatropha

### Technique analytique :

Pour évaluer la détoxification des tourteaux dans la présente description de l'invention, γ-compris dans les exemples, la préparation des échantillons ainsi que le dosage des esters de phorbol ont été réalisés selon la méthode de Makkar (Makkar HPS, Becker K, Sporer F, Wink M (1997) Studies on nutritive potential and toxic constituents of différent provenances of Jatropha curcas. J Agric Food Chem 45:3152-3157).

### Préparation des échantillons :

Les échantillons liquides sont dilués dans le méthanol puis injectés. Pour les échantillons solides, les esters de phorbol sont tout d'abord extraits au pilon et au mortier avec du méthanol, puis les extraits alcooliques sont injectés.

### Conditions opératoires :

Détecteur : barrette diode (intégration des pics à 280nm).
Colonne : C18 phase inverse (LiChrospher 100, 5µm), 250 x 4 mm + pré-colonne.
Four : 22°C (T amb).
Eluants : B = Eau acidifiée (1,75ml H3P04 (85%) dans 1L d'H2O)
A = Acétonitrile

**Tableau 9 : Gradients**

| | Eau acidifiée B (%) | Acétonitrile A (%) |
|---|---|---|
| 0 à 1 min | 60 | 40 |
| 1 à 10 min | 50 | 50 |
| 10 à 40 min | 25 | 75 |
| 40 à 55 min | 0 | 100 |
| 55 à 70 min | 0 | 100 |

Débit : 1,3ml/min.

### Dosage des esters de phorbol dans le tourteau de première pression produit à l'exemple 1 :

**Tableau 10 : Dosage des esters de phorbol du tourteau de jatropha**

| | Teneur en EP mg/g | Répartition des EP (en % des EP de la graine) |
|---|---|---|
| Graine engagée (3) | **3,5** | 100 |
| Tourteau gras | **2,4** | **50,1** |

Le tourteau de première pression demeure encore très concentré en esters de phorbol (EP), soit l'équivalent de 50% des EP de la graine de départ.

### Dosage des esters de phorbol dans le tourteau issu du procédé de trituration réactive et produit à l'exemple 4 :

**Tableau 11 : Répartition des esters de phorbol dans l'essai de trituration réactive - Essai 10E51 (essai de trituration réactive avec co-solvant méthanol hexane 50/50)**

| | Teneur en EP mg/g | Répartition des EP (en % des EP de la graine) |
|---|---|---|
| Tourteau (1) | **0,1** | **1,7** |

| | | |
|---|---|---|
| (1) Température séchage = 100°C, 16 heures. | | |

Le tourteau issu du procédé selon l'invention de trituration réactive sur tourteau pression avec co-solvant est beaucoup plus détoxifié que celui issu d'un procédé de première pression. Sa teneur en esters de phorbol (EP), soit 0,1 mg/g de tourteau, correspond en effet à 1,7% des EP de la graine. Le procédé de trituration réactive réalisé sur un tourteau de première pression est bien un procédé détoxifiant.

## Revendications

1. Procédé comprenant au moins une étape v) de trituration réactive consistant à mettre en contact un tourteau gras comprenant de 3 à 30% d'huile avec un alcool léger anhydre et un catalyseur alcalin dans des conditions de température et de durée suffisantes pour permettre l'extraction et la transestérification de l'huile végétale et conduisant à l'obtention d'un mélange comprenant des esters d'acides gras et du glycérol, et un tourteau déshuilé comprenant moins de 3% d'huile, dans lequel l'étape v) de trituration comprend l'introduction simultanée dans un réacteur contenant ledit tourteau, de l'alcool léger anhydre, du catalyseur basique, et en outre d'un co-solvant sélectionné dans le groupe : hexane, heptane, benzène, bicyclohexyle, cyclohexane, décaline, décane, essence, éther de pétrole, kérosène, kerdane, gazole, pétrole lampant, Méthylcyclohexane, Naphta (Texsolve V), Skellite, Tetradécane, Texsolve (B, C, H, S, S-2, S-66, S-LO, V), le CO2 supercritique, le propane ou le butane pressurisés, les solvants naturels tels que les terpènes (limonène, alpha et béta pinène)), des éthers tels que le diméthyléther, le diéthyléther, des cétones tels que l'acétone et des mélanges de tous ces solvants.

2. Procédé selon la revendication 1, comprenant en outre, avant l'étape v) de trituration, au moins une étape ii) de pressage de graines de plantes oléagineuses pour récupérer une partie de l'huile des graines et fabriquer ledit tourteau gras.

3. Procédé selon la revendication 1 ou 2, comprenant en outre, avant l'étape ii), au moins une des étapes i) suivantes : concassage, décorticage, préchauffage et/ou séchage des graines à une température inférieure ou égale à 100°C.

4. Procédé selon l'une quelconque des revendications précédentes comprenant en outre, une étape iii) de floconnage du tourteau gras avant l'étape v) de trituration réactive.

5. Procédé selon l'une quelconque des revendications précédentes comprenant en outre, une étape iv) de séchage du tourteau gras à une température comprise dans la gamme de 60 à 140°C, avant l'étape v) de trituration réactive.

6. Procédé selon l'une quelconque des revendications 2 à 5, dans lequel il s'écoule, entre l'étape ii) de fabrication du tourteau gras et l'étape v) de trituration réactive, moins de 72 heures, de préférence moins de 48 heures, de préférence moins de 24 heures, de préférence moins de 12 heures.

7. Procédé selon l'une quelconque des revendications 2 à 6 dans lequel les étapes sont réalisées en continu.

8. Procédé selon l'une quelconque des revendications précédentes dans lequel le tourteau gras a un taux d'acidité inférieur à 10 mg KOH/g, de préférence inférieur à 5 mg KOH/g, de préférence inférieur à 2 mg KOH/g.

9. Procédé selon l'une quelconque des revendications précédentes dans lequel l'alcool léger est le méthanol.

10. Procédé selon l'une quelconque des revendications précédentes dans lequel le catalyseur alcalin est la soude.

11. Procédé selon l'une quelconque des revendications précédentes dans lequel le rapport massique catalyseur/alcool/tourteau gras est compris dans la gamme 0,001 à 0,01/0,1 à 10/1, de préférence de 0,001 à 0,01/0,1 à 5/1, de préférence dans la gamme de 0,002 à 0,01/0,1 à 3/1, de manière encore plus préférée dans la gamme de 0,002 à 0,01/0,1 à 2/1.

12. Procédé selon l'une quelconque des revendications 1 à 11, dans lequel le rapport alcool léger / co-solvant est compris dans la gamme de 10/90 à 90/10.

13. Procédé selon l'une quelconque des revendications précédentes dans lequel le mélange comprenant des esters d'acides gras et du glycérol est soumis à une étape de décantation permettant d'obtenir une phase supérieure composée majoritairement d'esters d'acide gras et une phase inférieure composée majoritairement de glycérine et d'eau.

14. Procédé selon la revendication 13, dans lequel ladite phase supérieure est soumise à une succession de réactions chimiques et/ou de séparations/purifications conduisant à l'obtention de biodiésel.

15. Procédé selon l'une quelconque des revendications précédentes, dans lequel le tourteau gras est issu du pressage de graines de ricin et lesdits esters d'acide gras comprennent l'ester d'acide ricinoléique.

16. Tourteau déshuilé détoxifié susceptible d'être obtenu par le procédé de l'une des revendications 1 à 15 présentant une teneur en esters de phorbol inférieure ou égale à 0,3 mg/g, de préférence inférieure à 0,2 mg/g.

17. Utilisation du tourteau déshuilé selon la revendication 16 dans l'alimentation animale, comme engrais, comme bio-charbon, comme biocarburant, comme fuel, comme bio-gaz.

## Patentansprüche

1. Verfahren, das mindestens einen Schritt v) der reaktiven Zerkleinerung umfasst, bei dem man einen Ölpresskuchen, der 3 bis 30% Öl umfasst, mit einem wasserfreien niederen Alkohol und einem alkalischen Katalysator unter Temperatur- und Zeitbedingungen in Kontakt bringt, die ausreichen, dass die Extraktion und die Umesterung des Pflanzenöls ermöglicht wird, und zum Erhalt eines Fettsäureester und Glycerin umfassenden Gemischs und eines entölten Presskuchens, der weniger als 3% Öl umfasst, führt, wobei man in dem Zerkleinerungsschritt v) in einen den Presskuchen enthaltenden Reaktor gleichzeitig den wasserfreien niederen Alkohol, den alkalischen Katalysator und außerdem ein Co-Lösungsmittel einbringt, das aus der Gruppe mit: Hexan, Heptan, Benzol, Bicyclohexyl, Cyclohexan, Decalin, Decan, Benzin, Petrolether, Kerosin, Kerdane, Dieselkraftstoff, Gasöl, Methylcyclohexan, Naphtha (Texsolve V), Skellite, Tetradecan, Texsolve (B, C, H, S, S-2, S-66, S-LO, V), überkritischem CO₂, unter Druck stehendem Propan oder Butan, natürlichen Lösungsmitteln, wie Terpenen (Limonen, alpha- und beta-Pinen), Ethern, wie Dimethylether, Diethylether, Ketonen, wie Aceton, und Gemischen sämtlicher dieser Lösungsmittel ausgewählt wird.

2. Verfahren nach Anspruch 1, das außerdem vor dem Zerkleinerungsschritt v) mindestens einen Schritt ii) des Pressens von Samen ölhaltiger Pflanzen umfasst, um einen Teil des Öls der Samen zu gewinnen und den Ölpresskuchen herzustellen.

3. Verfahren nach Anspruch 1 oder 2, das außerdem vor dem Schritt ii) mindestens einen der folgenden Schritte i) umfasst: Aufbrechen, Schälen, Vorerhitzen und/oder Trocknen der Samen bei einer Temperatur von weniger als oder gleich 100°C.

4. Verfahren nach einem der vorhergehenden Ansprüche, das außerdem einen Schritt iii) des Flockierens des Ölpresskuchens vor dem Schritt der reaktiven Zerkleinerung v) umfasst.

5. Verfahren nach einem der vorhergehenden Ansprüche, das außerdem einen Schritt iv) des Trocknens des Ölpresskuchens bei einer Temperatur im Bereich von 60 bis 140°C vor dem Schritt v) der reaktiven Zerkleinerung umfasst.

6. Verfahren nach einem der Ansprüche 2 bis 5, wobei zwischen dem Schritt ii) der Herstellung des Ölpresskuchens und dem Schritt v) der reaktiven Zerkleinerung weniger als 72 Stunden, vorzugsweise weniger als 48 Stunden, bevorzugt weniger als 24 Stunden, vorzugsweise weniger als 12 Stunden vergehen.

7. Verfahren nach einem der Ansprüche 2 bis 6, wobei die Schritte im Durchlaufverfahren durchgeführt werden.

8. Verfahren nach einem der vorhergehenden Ansprüche, wobei der Ölpresskuchen eine Säurezahl unter 10 mg KOH/g, vorzugsweise unter 5 mg KOH/g, bevorzugt unter 2 mg KOH/g besitzt.

9. Verfahren nach einem der vorhergehenden Ansprüche, wobei der niedere Alkohol Methanol ist.

10. Verfahren nach einem der vorhergehenden Ansprüche, wobei der alkalische Katalysator Natriumhydroxid ist.

11. Verfahren nach einem der vorhergehenden Ansprüche, wobei das Massenverhältnis Katalysator/Alkohol/Ölpresskuchen im Bereich von 0,001 bis 0,01/0,1 bis 10/1, vorzugsweise von 0,001 bis 0,01/0,1 bis 5/1, bevorzugt im Bereich von 0,002 bis 0,01/0,1 bis 3/1, noch stärker bevorzugt im Bereich von 0,002 bis 0,01/0,1 bis 2/1 liegt.

12. Verfahren nach einem der Ansprüche 1 bis 11, wobei das Verhältnis niederer Alkohol/Co-Lösungsmittel im Bereich von 10/90 bis 90/10 liegt.

13. Verfahren nach einem der vorhergehenden Ansprüche, wobei das Fettsäureester und Glycerin enthaltende Gemisch einem Dekantierungsschritt unterzogen wird, der es gestattet, eine obere Phase, die größtenteils aus Fettsäureestern besteht, und eine untere Phase, die größtenteils aus Glycerin und Wasser besteht, zu erhalten.

14. Verfahren nach Anspruch 13, wobei die obere Phase einer Abfolge von chemischen Reaktionen und/oder Trennungen/Reinigungen unterzogen wird, die zur Herstellung von Biodiesel führen.

15. Verfahren nach einem der vorhergehenden Ansprüche, wobei der Ölpresskuchen aus dem Pressen von Rizinuskapseln erhalten wird und die Fettsäureester Rizinolsäureester umfassen.

16. Entgifteter entölter Presskuchen, der durch das Verfahren nach einem der Ansprüche 1 bis 15 erhältlich ist und einen Gehalt an Phorbolestern von weniger als oder gleich 0,3 mg/g, vorzugsweise weniger als oder gleich 0,2 mg/g aufweist.

17. Verwendung des entölten Presskuchens nach Anspruch 16 in Tierfutter, als Dünger, als Biokohlenstoff, als Biokraftstoff, als Brennstoff, als Biogas.

## Claims

1. Process comprising at least one reactive trituration step v) which consists in placing an oil cake comprising from 3% to 30% oil in contact with an anhydrous light alcohol and an alkaline catalyst under temperature and time conditions that are sufficient to allow the extraction and transesterification of the plant oil and leading to the production of a mixture comprising fatty acid esters and glycerol, and a de-oiled cake comprising less than 3% oil, in which the trituration step v) comprises the simultaneous introduction into a reactor containing the said cake, of anhydrous light alcohol, of basic catalyst and also of a cosolvent selected from the group: hexane, heptane, benzene, bicyclohexyl, cyclohexane, decalin, decane, light spirit, petroleum ether, kerosene, kerdane, diesel oil, lamp oil, methylcyclohexane, naphtha (Texsolve V), skellite, tetradecane, Texsolve (B, C, H, S, S-2, S-66, S-LO, V), supercritical CO₂, pressurized propane or butane, natural solvents such as terpenes (limonene, alpha and beta pinene), ethers such as dimethyl ether or diethyl ether, ketones such as acetone, and mixtures of all these solvents.

2. Process according to Claim 1, also comprising, before the trituration step v), at least one step ii) of pressing of seeds of oil-yielding plants to recover part of the oil from the seeds and to manufacture the said oil cake.

3. Process according to Claim 1 or 2, also comprising, before step ii), at least one of the following steps i): crushing, hulling, preheating and/or drying of the seeds at a temperature of less than or equal to 100°C.

4. Process according to any one of the preceding claims, also comprising a step iii) of flaking the oil cake before the reactive trituration step v).

5. Process according to any one of the preceding claims, also comprising a step iv) of drying the oil cake at a temperature in the range from 60 to 140°C, before the reactive trituration step v).

6. Process according to any one of Claims 2 to 5, in which less than 72 hours, preferably less than 48 hours, preferably less than 24 hours and preferably less than 12 hours pass between step ii) of manufacture of the oil cake and the reactive trituration step v).

7. Process according to any one of Claims 2 to 6, in which the steps are performed continuously.

8. Process according to any one of the preceding claims, in which the oil cake has an acidity level of less than 10 mg KOH/g, preferably less than 5 mg KOH/g and preferably less than 2 mg KOH/g.

9. Process according to any one of the preceding claims, in which the light alcohol is methanol.

10. Process according to any one of the preceding claims, in which the alkaline catalyst is sodium hydroxide.

11. Process according to any one of the preceding claims, in which the catalyst/alcohol/oil cake mass ratio is in the range 0.001 to 0.01/0.1 to 10/1, preferably from 0.001 to 0.01/0.1 to 5/1, preferably in the range from 0.002 to 0.01/0.1 to 3/1 and even more preferably in the range from 0.002 to 0.01/0.1 to 2/1.

12. Process according to any one of Claims 1 to 11, in which the light alcohol/cosolvent ratio is in the range from 10/90 to 90/10.

13. Process according to any one of the preceding claims, in which the mixture comprising fatty acid esters and glycerol is subjected to a decantation step to obtain an upper phase predominantly composed of fatty acid esters and a lower phase predominantly composed of glycerol and water.

14. Process according to Claim 13, in which the said upper phase is subjected to a succession of chemical reactions and/or of separations/purifications leading to the production of biodiesel.

15. Process according to any one of the preceding claims, in which the oil cake is obtained from the pressing of castor oil seeds and the said fatty acid esters comprise ricinoleic acid ester.

16. Detoxified de-oiled cake which may be obtained via the process of one of Claims 1 to 15, having a content of phorbol esters of less than or equal to 0.3 mg/g and preferably less than 0.2 mg/g.

17. Use of the de-oiled cake according to Claim 16 in animal feed, as fertilizer, as biocarbon, as biofuel, as fuel or as biogas.
